# FASCICULE DE BREVET EUROPEEN

(11) **EP 1 732 565 B1**
(45) Date de publication et mention de la délivrance du brevet: **09.03.2016**
(21) Numéro de dépôt: 05755780.3
(22) Date de dépôt: 01.04.2005
(51) Int. Cl.: A61K 31/5377, A61P 9/10

(54) **COMPOSITION ORALE DE LA MOLSIDOMINE A LIBERATION PROLONGEE POUR LE TRAITEMENT DE L"ATHEROSCLEROSE**
ORALE MOLISDOMIN-ZUSAMMENSETZUNG MIT VERZÖGERTER FREISETZUNG ZUR BEHANDLUNG VON ARTERIOSKLEROSE
SUSTAINED-RELEASE ORAL MOLSIDOMINE COMPOSITION FOR TREATING ATHEROSCLEROSIS

(30) Priorité: 05.04.2004 FR 0403534
(43) Date de publication de la demande: 20.12.2006
(73) Titulaire: Therabel Pharmaceuticals Limited, Loughrea, C. Galway (IE)
(72) Inventeur: GECZY, Jozsef-Michel, B-1180 Bruxelles (BE)
(74) Mandataire: Hubert, Philippe
(86) Numéro de dépôt international: PCT/EP2005/003531
(87) Numéro de publication internationale: WO 2005/107761

(56) Documents cités:
- WO-A-01/62256
- US-A- 5 385 937
- US-B1- 6 472 390
- DATABASE BIOSIS [Online] BIOSCIENCES INFORMATION SERVICE, PHILADELPHIA, PA, US; 1991, GRODZINSKA L ET AL: "THERAPEUTIC EFFECTS OF MOLSIDOMINE NO-DONOR IN PATIENTS WITH ATHEROSCLEROSIS OBLITERANS OF THE LOWER LIMBS" XP002304305 Database accession no. PREV199293044329 & JOURNAL OF DRUG DEVELOPMENT, vol. 4, no. 1, 1991, pages 39-46, ISSN: 0952-9500
- ROLLAND P H ET AL: "Local delivery of NO-donor molsidomine post-PTA improves haemodynamics, wall mechanics and histomorphometry in atherosclerotic porcine SFA." EUROPEAN JOURNAL OF VASCULAR AND ENDOVASCULAR SURGERY : THE OFFICIAL JOURNAL OF THE EUROPEAN SOCIETY FOR VASCULAR SURGERY. MAR 2002, vol. 23, no. 3, mars 2002 (2002-03), pages 226-233, XP009039226 ISSN: 1078-5884
- DATABASE EMBASE [Online] ELSEVIER SCIENCE PUBLISHERS, AMSTERDAM, NL; 15 septembre 2002 (2002-09-15), BULT H: "Nitric oxide related interventions in atherosclerosis" XP002304306 Database accession no. EMB-2002345488 & TIJDSCHRIFT VOOR GENEESKUNDE 15 SEP 2002 BELGIUM, vol. 58, no. 18, 15 septembre 2002 (2002-09-15), pages 1181-1188, ISSN: 0371-683X
- DATABASE WPI Section Ch, Week 200377 Derwent Publications Ltd., London, GB; Class B02, AN 2003-826115 XP002304307 & KR 2003 039 981 A (UNIV INJE) 22 mai 2003 (2003-05-22)
- ADAMS M R ET AL: "L-arginine reduces human monocyte adhesion to vascular endothelium and endothelial expression of cell adhesion molecules." CIRCULATION. 4 FEB 1997, vol. 95, no. 3, 4 février 1997 (1997-02-04), pages 662-668, XP002304303 ISSN: 0009-7322
- TAKAHASHI MASAFUMI ET AL: "Nitric oxide attenuates adhesion molecule expression in human endothelial cells" CYTOKINE, vol. 8, no. 11, 1996, pages 817-821, XP002304304 ISSN: 1043-4666

## Description

La présente invention a pour objet une nouvelle utilisation thérapeutique de la molsidomine et de ses sels pharmaceutiquement acceptables, sous une forme galénique orale à libération prolongée, efficace pendant 24 heures, dans la prévention ou le traitement de l'athérosclérose.

L' athérosclérose est une affection progressive des artères qui compromet l'irrigation sanguine des organes situés en aval. Ainsi, les plaques d'athérome qui apparaissent sur la paroi des artères coronaires peuvent entraîner une restriction de l'irrigation du coeur (ischémie) pouvant conduire à un infarctus du myocarde, principale cause de décès dans les pays industrialisés.

Des facteurs de risque tels que l'hypercholestérolémie ou l'hypertension stimulent la formation de lésions athérosclérotiques.

Dans le cas de l'hypercholestérolémie, le mécanisme conduisant à la formation de ces lésions peut être résumé comme suit.

Des lipoprotéines de basse densité (généralement dénommées LDL) s'accumulent dans l'intima (paroi intérieure de l'artère) où elles sont oxydées en Ox-LDL.

La présence de ces molécules oxydées dans l'intima entraînent la synthèse et l'expression de molécules d'adhésion telles que l'ICAM-1 (intercellular adhésion molecule-1) et le VCAM-1 (vascular cell adhésion molecule-1) à la surface de l'endothélium.

Ces molécules d'adhésion ont la propriété de fixer les monocytes provenant de la circulation sanguine, lesquels s'infiltrent dans la paroi vasculaire et se transforment en macrophages.

Ces macrophages absorbent les Ox-LDL (phagocytose) et se transforment ainsi lentement en cellules spumeuses saturées de lipides. Par ailleurs, ces macrophages libèrent des cytokines qui provoquent une réaction inflammatoire locale, laquelle favorise un recrutement encore plus important de monocytes.

Les cellules musculaires lisses se multiplient et migrent dans l'intima. Elles y sécrètent du collagène, des fibres élastiques et des protéoglycanes conduisant à un épaississement progressif des lésions.

Les cellules spumeuses synthétisent aussi un facteur tissulaire qui participe au dépôt de fibrines dans la plaque athéromateuse. Il en résulte des lésions endothéliales de plus en plus sévères.

Il résulte de ce qui précède que les molécules d'adhésion et en particulier l'ICAM-1 interviennent dans le processus de formation des lésions athérosclérotiques.

En effet, l'ICAM-1 est fortement surexprimé sur l'endothélium recouvrant les plaques athéromateuses dans les artères coronaires et les carotides humaines.

Des études cliniques récentes indiquent que la sévérité de l'athérosclérose est corrélée avec le taux d'ICAM-1 soluble (ci-après ICAM-1s). Par ailleurs, dans des modèles athérosclérotiques de souris, on a observé que la déplétion en ICAM-1s conduit à un rôle protecteur contre la progression de l'athérosclérose.

De ce fait, l'abaissement des taux d'ICAM-1 soluble semble constituer un moyen efficace pour restaurer les fonctions endothéliales normales et pour prévenir et/ou ralentir la progression de l'athérosclérose.

On sait, par ailleurs, que la molsidomine est un composé particulièrement utile dans le traitement préventif de la crise angineuse sous toutes ses formes, qui agit en provoquant une relaxation de la fibre musculaire lisse vasculaire, et une inhibition des phases précoces de l'activation plaquettaire. L'étude publiée par Grodzinska et al. dans J. Drug Dev. 1991, 4(1), 39-46, met en évidence les propriétés vasodilatatrices de la molsidomine dans le traitement des symptômes liés à la condition athérosclérotique.

La molsidomine a d'abord été commercialisée essentiellement :
- sous forme de comprimés sécables à libération immédiate dosés à 2 mg et 4 mg, généralement administrés trois fois par jour dans le traitement de l'angor d'effort, et quatre fois par jour dans le traitement de l'angor de repos et de l'angor d'effort sévère ; puis
- sous forme de comprimés à libération prolongée dosés à 8 mg, destinés à être administrés deux fois par jour pour un traitement prophylactique et au long cours de l'angine de poitrine.

Plus récemment, la molsidomine a été commercialisée (notamment sous la dénomination Coruno® en Belgique) sous la forme d'une composition orale solide à libération prolongée, efficace pendant 24 heures et dosée à 16 mg, pour la prévention et le traitement chronique au long cours de l'angine de poitrine stable. L'efficacité et la tolérance de cette composition ont été démontrées chez un grand nombre de patients dans des études de courtes et de longues durées.

C'est dans ce contexte qu'il a été découvert, de façon surprenante et tout à fait inattendue, que la molsidomine, sous la forme d'une composition orale solide à libération prolongée, efficace pendant 24 heures, permettait de restaurer les fonctions endothéliales et de ce fait de prévenir les processus physiopathologiques conduisant à l'athérosclérose, et/ou d'en ralentir la progression.

Plus précisément, il a été découvert que l'administration journalière d'une telle forme galénique de molsidomine entraîne une diminution sensible de la quantité d'ICAM-1 soluble circulant, lequel est considéré comme un biomarqueur de l'athérosclérose et qu'ainsi, outre ses propriétés anti-angineuses connues, la molsidomine permet d'inhiber la fixation des monocytes sur l'endothélium et par conséquent d'assurer la restauration des fonctions endothéliales, et de prévenir et/ou ralentir la progression de l'athérosclérose.

Ainsi, selon un premier aspect, la présente invention a pour objet l'utilisation de la molsidomine ou de l'un de ses sels pharmaceutiquement acceptables, telle que définie dans les revendications annexées.

Dans le cadre de la présente invention, la molsidomine peut être utilisée sous forme libre mais également sous la forme d'un sel pharmaceutiquement acceptable, tel qu'en particulier un chlorhydrate.

Dans la description qui va suivre, on utilisera le terme molsidomine pour désigner aussi bien la forme libre que la forme salifiée de cette molécule.

L'expression "efficace(s) pendant 24 heures" utilisée ici signifie que la quantité de molsidomine libérée par la forme pharmaceutique utilisée est suffisante pour conduire à une concentration plasmatique thérapeutique d'au moins 5 ng/ml, et de préférence d'au moins 10 ng/ml de plasma, pendant une période d'environ 24 heures.

Les effets de la molsidomine dans le traitement de l'athérosclérose sont particulièrement importants dans le cadre d'un traitement d'une longue durée (6 mois au moins). Ces effets sont particulièrement remarquables sur des patients angineux stables.

Des résultats particulièrement intéressants dans le traitement de l'athérosclérose ont été obtenus selon l'invention par l'administration de comprimés à libération prolongée dosés à 16 mg correspondant à la spécialité pharmaceutique Coruno® commercialisée en Belgique.

Cette forme galénique et son procédé de fabrication ont été décrits dans la demande de brevet internationale WO 01/62256.

D'une façon générale, les formes galéniques de molsidomine décrites dans cette demande de brevet internationale sont essentiellement caractérisées en ce qu'elles présentent un taux de dissolution in vitro [mesuré spectrophotométriquement à 286 ou 311 nm selon la méthode décrite dans la Pharmacopée Européenne, 3ème édition (ou U.S.P. XXIV) à 50 t.p.m. dans 500 ml d'un milieu HCl 0,1 N, à 37°C] de :
- 15 à 25 % de molsidomine libérée après 1 heure
- 20 à 35 % de molsidomine libérée après 2 heures
- 50 à 65 % de molsidomine libérée après 6 heures
- 75 à 95 % de molsidomine libérée après 12 heures
- > 85 % de molsidomine libérée après 18 heures
- > 90 % de molsidomine libérée après 24 heures,
le pic plasmatique de molsidomine obtenu in vivo se présentant dans les 2,5 à 5 heures, de préférence dans les 3 à 4 heures, suivant l'administration de ladite forme et ayant une valeur comprise entre 25 et 40 ng/ml de plasma.

Dans ce contexte, " le pic plasmatique de molsidomine obtenu in vivo " correspond à la concentration maximale moyenne de molsidomine trouvée dans le plasma d'au moins 10 volontaires en bonne santé.

Dans le cadre de la présente invention, toute forme galénique telle que décrite dans cette demande de brevet internationale WO 01/62256 peut être avantageusement utilisée.

D'une façon générale, ces formes galéniques permettent l'administration de molsidomine à des doses journalières comprises entre 14 et 24 mg, et de préférence entre 16 et 20 mg.

De telles formes galéniques de mosidomine, présentant un profil de libération sur 24 heures caractérisé par une absence de pics rapprochés et de vallées trop marquées, s'avèrent particulièrement adaptées dans le traitement de l'athérosclérose, en garantissant une libération constante et stable de la molsidomine sur les sites touchés par l'athérosclérose.

Par conséquent, la libération relativement lente et constante de molsidomine sans pics plasmatiques marqués et rapprochés semble constituer une caractéristique importante pour obtenir l'effet recherché dans le traitement de l'athérosclérose.

L'utilisation de la molsidomine sous ces formes galéniques à libération prolongée est particulièrement intéressante dans la mesure où ce composé n'induit pas de tolérance et que sa sécurité d'emploi a été démontrée chez un grand nombre de patients.

### Mise en évidence des effets de la molsidomine dans le traitement de l'athérosclérose

### 1. Caractéristiques de la population étudiée et schéma expérimental

Les effets favorables de la molsidomine dans le traitement de l'athérosclérose ont été mis en évidence par une étude clinique à long terme réalisée sur 172 patients présentant de l'angine de poitrine stable.

Cette étude a comporté 3 phases consécutives :
- une pré-étude de 7 jours sous placebo ;
- une étude croisée, randomisée, double-aveugle et double-placebo de 4 semaines au cours de laquelle les patients ont reçu en alternance (2 fois 2 semaines) une formulation à base de molsidomine dosée à 8 mg actuellement commercialisée en Belgique sous la dénomination Corvatard® (2 prises journalières) et une formulation à base de molsidomine dosée à 16 mg et efficace pendant 24 heures, actuellement commercialisée en Belgique sous la dénomination Coruno® (1 prise journalière) ;
- une étude de 12 mois pendant laquelle les 172 patients ont reçu une formulation à base de molsidomine dosée à 16 mg actuellement commercialisée en Belgique sous la dénomination Coruno^{®}.

Cette étude peut être représentée schématiquement de la façon suivante.

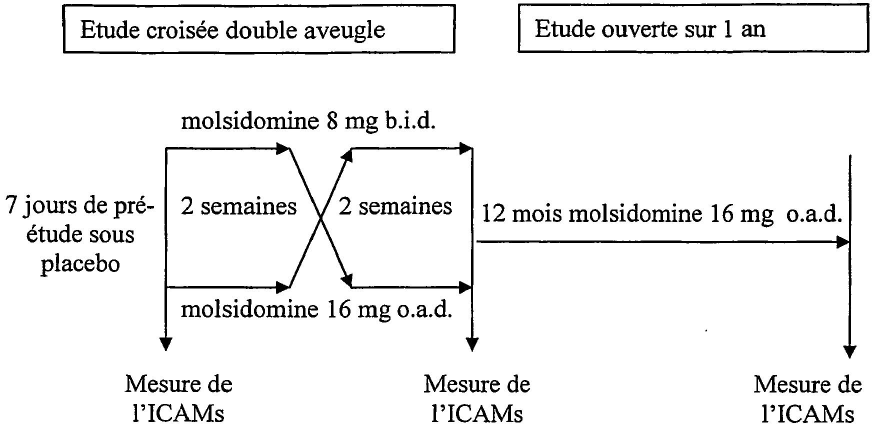

Dans ce schéma, et dans la description qui va suivre, l'abréviation "b.i.d" (pour "bis in die") est utilisée pour caractériser l'administration de la molsidomine 8 mg en deux prises journalières et l'abréviation "o.a.d" (pour "once a day") est utilisée pour caractériser l'administration de la molsidomine 16 mg en une prise journalière.

Il est à noter que durant la seconde phase de l'étude, l'utilisation concomitante d'autres médicaments anti-angineux était interdite, à l'exception du dinitrate d'isosorbide sublingual (s.l.) (ISDN) 5 mg en comprimés qui pouvait être consommé ad libitum pour soulager les symptômes de douleur angineuse.

Par ailleurs, durant la troisième phase de l'étude, l'utilisation concomitante de béta-bloquants et/ou d'antagonistes calciques était autorisée, tandis que l'utilisation de nitrés oraux et du sildenafil était toujours interdite.

La molsidomine 16 mg o.a.d. devait être prise oralement chaque matin pendant un an.

L'étude a été entreprise selon les Directives sur la recherche clinique pour les médicaments anti-angineux fournis par le CPMP, selon les bonnes pratiques cliniques (étape 4) telles que mises en application dans la Communauté européenne.

Au cours de cette étude, on a noté la fréquence hebdomadaire des crises angineuses et la fréquence hebdomadaire de consommation de comprimés d'ISDN s.l. 5 mg.

Par ailleurs et surtout, les concentrations d'ICAM-1s ont été mesurées à l'issue de chacune des trois phases de l'étude, la première mesure constituant la ligne de base.

Plus précisément, les concentrations d'ICAM-1s ont été mesurées de la façon suivante.

Des échantillons de sang (5 ml) ont été prélevés dans des tubes non-héparinisés. Ces échantillons ont été conservés à température ambiante, puis ils ont été centrifugés. Le sérum a été séparé et immédiatement congelé à - 20°C jusqu'à l'analyse.

L'ICAM-1 circulant a été mesuré en utilisant un test ELISA disponible dans le commerce (R&D Systems Europe).

### 2. Analyses statistiques

Des statistiques descriptives (moyennes, écart-type et %) ont été utilisées pour caractériser la démographie et les autres paramètres de la population des patients de l'étude.

Des analyses de la variance pour mesures répétées, avec le temps comme critère de classification, suivies par des tests de Bonferroni post-hoc quand elles étaient significatives, ont été utilisées pour évaluer l'évolution de la fréquence hebdomadaire des crises angineuses, de la consommation hebdomadaire de comprimés nitrés s.l. et des taux d`ICAMs circulants pendant les parties à court terme (deuxième phase) et à long terme (troisième phase) de l'étude.

Des tests t de Student ou des ANOVA à un critère de classification ont été utilisés pour évaluer les effets du sexe, de la consommation d'alcool, des habitudes tabagiques et de l'utilisation concomitante de médicaments sur les taux dICAMs mesurés à l'issue de chaque phase de l'étude (ligne de base pré-étude sous placebo, après le traitement de quatre semaines et après le traitement d'une année).

Des analyses de la variance pour mesures répétées ont été utilisées pour évaluer les effets des mêmes facteurs de risque sur l'évolution des taux d'ICAM-1s.

Des coefficients de corrélation de Pearson ont été calculés pour détecter des liens possibles entre les taux d'ICAM-1s et les variables démographiques continues ou les facteurs de risque. La même méthode a été employée pour évaluer les corrélations entre les variations du taux d'ICAM-1s et les changements des facteurs de risque.

Par ailleurs, on a déterminé les quatre quartiles des changements de taux d'ICAM-1s après une année de traitement. Un test ANOVA à un critère de classification suivi, quand il est significatif, de tests de Bonferroni post-hoc ajustés, a été utilisé pour déterminer les effets des changements de taux d'ICAM-1s, dans les 4 catégories ainsi définies (4 quartiles) sur les changements observés (de la ligne de base à la fin du traitement d'une année) dans la fréquence hebdomadaire des crises angineuses et de consommation de comprimés d'ISDN.

### 3. Résultats

Les caractéristiques démographiques de la population à la ligne de base (issue de la pré-étude sous placebo), sont détaillées dans le tableau 1.

**Tableau 1. Caractéristiques démographiques et ligne de base des patients ayant participé à l'étude**

| | **N** | **Moyenne ± ET ou %** |
|---|---|---|
| Age (années) | 172 | 56,2 ± 8,3 |
| Durée de l'angine (années) | 172 | 4,4 ± 4,5 |
| Poids (kg) | 172 | 77,7 ± 12,0 |
| Fréquence hebdomadaire des crises angineuses | 107 | 3,7 ± 3,8 |
| Fréquence hebdomadaire de la consommation de comprimés d'ISDN | 107 | 2,5 ± 3,2 |
| Pression sanguine diastolique (mmHg) | 172 | 82,0 ± 8,0 |
| Pression sanguine systolique (mmHg) | 172 | 131,2 ± 15,1 |
| Rythme cardiaque (battements/min) | 172 | 76,6 ± 11,0 |
| ICAM-1s (ng/ml) | 172 | 272 ± 92 |
| Sexe | 172 | |
| Hommes | 117 | 68,0 |
| Femmes | 55 | 32,0 |
| Habitudes tabagiques | 172 | |
| Non-fumeurs | 81 | 47,1 |
| Anciens fumeurs | 59 | 34,3 |
| Fumeurs | 32 | 18,6 |
| Traitement concomitant | 172 | |
| Pas d'autre médication anti-angineuse | 116 | 67,4 |
| Béta-bloquants | 50 | 29,1 |
| Antagoniste calcique | 2 | 1,2 |
| Les deux médicaments | 4 | 2,3 |
| Consommation d'alcool | 172 | |
| < 1 verre/jour | 161 | 93,6 |
| >= 1 verre/jour | 11 | 6,4 |

| | | |
|---|---|---|
| ET = écart-type | | |

Comme le montre le tableau 1, les patients de l'étude étaient âgés de 56,2 ± 8,3 ans (moyenne ± écart-type), mâles en majorité (68,0 %) et avaient souffert d'angine de poitrine stable durant 4,4 ± 4,5 ans en moyenne.

Pendant la première phase de l'étude sous placebo, précédant les traitements actifs, les fréquences hebdomadaires de crises angineuses et de consommation de comprimés d'ISDN étaient respectivement de 3,7 ± 3,8 crises/semaine et 2,5 ± 3,2 comprimés/semaine.

Bien que la consommation concomitante de médicaments anti-angineux ait été autorisée pendant cette phase de l'étude, 67,4 % de patients n'ont pris aucun autre médicament que la molsidomine et 29,1 % des patients n'ont pris que des béta-bloquants.

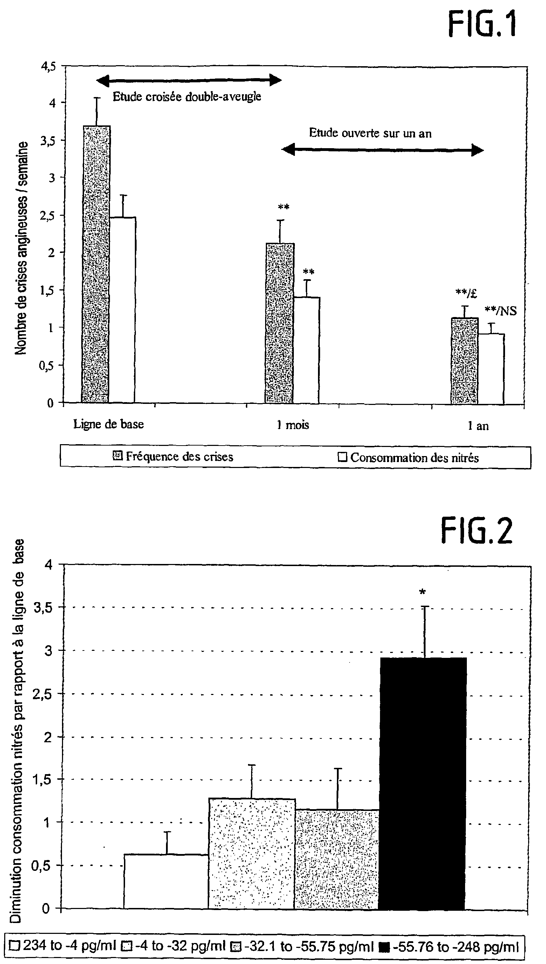

On a représenté à la figure 1 l'évolution de la fréquence hebdomadaire des crises angineuses et de la consommation de comprimés de nitrés sublinguaux pendant les deuxième et troisième phases de l'étude.

Les résultats sont présentés sous forme de moyennes ± erreurs sur le moyenne (SEM); ANOVA pour mesures répétées, p<0,0001; tests de Bonferroni post-hoc : ** comparaisons versus ligne de base, p<0,0001; £ comparaison entre 4 semaines et un an, p=0,002; NS comparaison entre 4 semaines et un an, p=0,105.

Comme le montre la figure 1, des diminutions globales significatives de la fréquence hebdomadaire des crises angineuses et de la consommation de comprimés nitrés s.l. ont été mesurées (p<0,0001; ANOVA) au cours de l'étude.

Pour la fréquence des crises angineuses, les différences étaient significatives entre la ligne de base et le traitement de 4 semaines (p<0,0001; Bonferroni) ainsi qu'entre la ligne de base et le traitement d'un an (p<0,0001; Bonferroni). Les différences étaient également significatives entre le traitement de 4 semaines et le traitement d'un an (p=0,002; Bonferroni).

Pour la consommation de comprimés nitrés s.l., les différences étaient significatives entre la ligne de base et le traitement de 4 semaines (p<0,0001; Bonferroni) ainsi qu'entre la ligne de base et le traitement d'un an (p<0,0001; Bonferroni).

On a présenté dans le tableau 2 ci-après, les concentrations d'ICAM-1s (ng/ml) mesurées pendant les parties à court terme (deuxième phase) et à long terme (troisième phase) de l'étude, afin de déterminer notamment l'influence du sexe, de la consommation d'alcool, des habitudes tabagiques et des médicaments concomitants, sur l'évolution des taux d'ICAM-1s circulants.

**Tableau 2. Evolution des concentrations d'ICAM-1s (ng/ml) pendant les parties à court terme et à long terme de l'étude et influence du sexe, de la consommation d'alcool, des habitudes tabagiques et des médicaments concomitants**

| | **N** | **Ligne de base pré-étude sous placebo** | **4-semaines double-aveugle** | **Un an ouvert** | **ANOVA Valeur p (effet du temps)** | **ANOVA Valeur p (interaction temps-groupe)** |
|---|---|---|---|---|---|---|
| | | **Moyenne ± ET** | **Moyenne + ET** | **Moyenne ± ET** | | |
| **Tous les patients** | 172 | 272 ± 92 | 274 ± 87 | 246 ± 99^{£/£} | <0,0001 | NA |
| **Sexe** | | | | | | |
| Hommes | 117 | 264 ±70 | 264 ± 58 | 236 ± 62^{£/£} | <0,0001 | 0,914 |
| Femmes | 55 | 291 ± 126 | 293 ± 128 | 267 ± 149^{**/£} | 0,001 | |
| **Consommation d'alcool** | | | | | | |
| < 1 verre/jour | 161 | 273 ± 94 | 276 ± 89 | 249 ± 102^{£/£} | <0,0001 | 0,149 |
| ≥ 1 verre/jour | 11 | 258 ± 63 | 242 ± 38 | 202 ± 25^{*/**} | 0,003 | |
| **Habitudes tabagiques** | | | | | | |
| Non-fumeurs | 81 | 265 ± 86 | 262 ±74 | 233 ± 70^{£/£} | <0,0001 | 0,192 |
| Anciens fumeurs | 59 | 270 ± 71 | 277 ± 67 | 243 ± 63^{***/£} | <0,0001 | |
| Fumeurs | 32 | 295 ± 133 | 297 ± 138 | 282 ± 180 | 0,337 | |
| **Médication concomitante** | | | | | | |
| Rien | 78 | 280 ± 117 | 276 ± 112 | 251 ± 132^{£/£} | <0,0001 | 0,598 |
| Statines | 38 | 268 ± 74 | 277 ±70 | 250 ± 79^{NS/***} | <0,0001 | |
| Béta-bloquants | 30 | 276 ±64 | 281 ± 58 | 243 ± 51^{£/£} | <0,0001 | |
| Les deux médicaments | 20 | 246 ± 51 | 247 ± 45 | 222 ± 36 | 0,115 | |

| | | | | | | |
|---|---|---|---|---|---|---|
| */* = probabilité statistique versus ligne de base / versus résultats à 4-semaines; tests de Bonferroni post-hoc * = p<0,05; ** = p<0,01; ***= p=0,001; £ = p<0,0001; NS = non significatif p>0,05; NA = non applicable ; ET = écart-type | | | | | | |

Comme le montre le tableau 2, le traitement de 4 semaines avec la molsidomine (16 mg o.a.d ou 8 mg b.i.d.) n'a eu aucun effet sur les taux d'ICAM-1s circulants.

Cependant après 12 mois de prise quotidienne de molsidomine 16 mg o.a.d., les taux d'ICAM-1s étaient sensiblement (p<0,0001) plus bas (environ 10 %), comparés aux valeurs de la ligne de base avant l'étude croisée.

Les taux d'ICAM-1s circulants tendaient à être plus élevés chez les femmes que chez les hommes. Cependant, l'interaction avec le sexe était non significative (p=0,914) par ANOVA, indiquant que la diminution d'ICAM-1s pendant le traitement d'une année avec la molsidomine était globalement parallèle pour les deux sexes.

Les consommateurs d'alcool avaient tendance à présenter des taux d'ICAM-1s plus bas que les non consommateurs d'alcool. Toutes les différences étaient non significatives et l'évolution du taux d'ICAM-1s pendant le traitement d'une année était globalement parallèle dans les deux groupes (p=0,149).

Les fumeurs avaient tendance à avoir des taux d'ICAM-1s plus élevés que les non-fumeurs ou que les anciens fumeurs mais à nouveau, l'évolution du taux d'ICAM-1s était globalement la même quelque soient les habitudes tabagiques (p=0,192).

L'utilisation de médicaments concomitants tels que les statines, les béta-bloquants ou les deux combinés n'avait aucune influence sur le taux d'ICAM-1s. Toutes les différences étaient non significatives et l'évolution du taux d'ICAM-1s était indépendante du type de médicament concomitant absorbé pendant la période d'une année de traitement (p=0,598).

À la ligne de base, il n'y avait aucune corrélation entre les concentrations d'ICAM-1s et les facteurs démographiques ou les facteurs de risque tels que l'âge (r = -0,068), le poids (r = -0,079), la durée de l'angine stable (r = 0,042), la fréquence hebdomadaire des crises angineuses (r = 0,137), la fréquence hebdomadaire de la consommation de comprimés d'ISDN s.l. (r = 0,124), la pression artérielle diastolique (r = 0,051), la pression artérielle systolique (r = 0,097) ou la fréquence cardiaque (r = 0,176).

Les mêmes conclusions pourraient être tirées en ce qui concerne les corrélations entre les variations de taux d'ICAM-1s et les changements des facteurs démographiques et des facteurs de risque après un traitement d'une année avec la molsidomine (données non présentées).

On a représenté à la figure 2, la diminution de la fréquence hebdomadaire de la consommation de comprimés d'ISDN en fonction de la variation du taux d'ICAM-1s circulant après un traitement d'une année avec la molsidomine 16 mg o.a.d. ; les 4 catégories des changements d'ICAM-1s correspondent aux 4 quartiles de la distribution.

Les résultats sont présentés sous forme de moyennes ± erreurs sur la moyenne (SEM); ANOVA p = 0,031; * tests de Bonferroni post-hoc p=0,038.

La figure 2, avec la distribution en quatre quartiles des changements des taux d'ICAM-1s pendant la troisième phase de l'étude montre que l'effet du changement du taux d'ICAM-1s sur l'évolution de la fréquence de la consommation de comprimés d'ISDN s.l. était significatif (p=0,031).

Les tests de Bonferroni post-hoc ont démontré que la diminution de la consommation d'ISDN entre le début et la fin de la troisième phase de l'étude (12 mois plus tard) était plus prononcée dans le groupe présentant la plus grande diminution d'ICAM-1s (4ème quartile de distribution) (p=0,038).

La même tendance a été mesurée pour le changement de la fréquence hebdomadaire des crises angineuses mais les différences entre les 4 quartiles des changements d'ICAM-1s étaient non significatives (p=0,072) (données non présentées).

### 4. Discussion

La présente étude a permis d'évaluer chez les patients présentant de l'angine de poitrine stable l'effet d'une courte période (4 semaines) et d'une longue période (une année) de traitement avec la molsidomine 16 mg o.a.d.

Les résultats obtenus montrent de façon surprenante qu'après une administration d'un an de cette forme galénique, les effets anti-angineux significatifs, déjà mesurés après un traitement de 4 semaines, persistent, et que les taux d'ICAM-1s circulants, marqueur pro-inflammatoire du dysfonctionnement endothélial et cible thérapeutique potentielle dans la pathologie de l'athérosclérose, sont sensiblement réduits.

Au début de l'étude, après une période pré-étude sous placebo de sept jours, les taux d'ICAM-1s étaient comparables aux valeurs obtenues dans d'autres études chez les patients souffrant d'une maladie coronaire cardiaque ou d'une angine stable. Les femmes et les fumeurs avérés avaient tendance à avoir des taux plus élevés que les hommes et les non-fumeurs ou anciens fumeurs, confirmant les observations d'études antérieures.

Le traitement à court terme (4 semaines) avec la molsidomine n'a entraîné aucun effet sur les taux d'ICAM-1 circulants. Chez ces patients présentant de l'angine stable, l'efficacité du traitement anti-angineux de 4 semaines était cependant significative étant donné que le nombre de crises angineuses et la consommation de nitrés sublinguaux ont diminué.

Après 12 mois de prise quotidienne de molsidomine 16 mg o.a.d., les taux d'ICAM-1s étaient sensiblement plus bas. Cette diminution était indépendante d'autres paramètres tels que le sexe, la consommation d'alcool, les habitudes tabagiques ou la médication concomitante. Après un an, les effets anti-angineux de la molsidomine étaient maintenus ou même améliorés et la diminution la plus prononcée de la consommation de nitrés sublinguaux a été observée chez les patients qui avaient la plus grande diminution des taux dICAM-1s (4ème quartile).

En conclusion, la réduction du marqueur ICAM-1s après un traitement quotidien d'un an avec la molsidomine 16 mg o.a.d. indique que ce composé, en plus de sa fonction anti-angineuse, favorise un état moins activé de l'endothélium et, de ce fait, permet de prévenir et/ou ralentir la progression de l'athérosclérose notamment chez les patients présentant de l'angine de poitrine stable.

## Revendications

1. Utilisation de la molsidomine ou de l'un de ses sels pharmaceutiquement acceptables, sous la forme d'une composition orale solide à libération prolongée efficace pendant 24 heures, pour la fabrication d'un médicament destiné à prévenir ou atténuer le développement de l'athérosclérose,
dans laquelle ladite composition présente un taux de dissolution in vitro, mesuré spectrophotométriquement à 286 ou 311 nm selon la méthode décrite dans la Pharmacopée Européenne, 3ème édition (ou U.S.P. XXIV) à 50 t.p.m. dans 500 ml d'un milieu HCl 0,1 N, à 37°C, de :
- 15 à 25 % de molsidomine libérée après 1 heure
- 20 à 35 % de molsidomine libérée après 2 heures
- 50 à 65 % de molsidomine libérée après 6 heures
- 75 à 95 % de molsidomine libérée après 12 heures
- > 85 % de molsidomine libérée après 18 heures
- > 90 % de molsidomine libérée après 24 heures,
le pic plasmatique de molsidomine obtenu in vivo se présentant dans les 2,5 à 5 heures, de préférence dans les 3 à 4 heures, suivant l'administration de ladite forme et ayant une valeur comprise entre 25 et 40 ng/ml de plasma,
dans laquelle la composition orale solide précitée comporte, par unité de dosage destinée à être administrée de façon journalière, entre 14 et 24 mg de molsidomine,
et dans laquelle le traitement est d'une durée d'au moins 6 mois.

2. Utilisation selon la revendication 1, **caractérisée en ce que** la composition orale solide précitée comporte, par unité de dosage destinée à être administrée de façon journalière, 16 mg de molsidomine.

3. Utilisation selon l'une des revendications 1 ou 2, **caractérisée en ce que** ladite composition orale solide précitée est administrée à des patients souffrants d'angine de poitrine.

## Patentansprüche

1. Verwendung von Molsidomin oder von einem seiner pharmazeutisch akzeptablen Salze, in Form einer festen oralen Zusammensetzung mit verlangsamter Freisetzung, die über 24 Stunden wirksam ist, für die Herstellung eines Medikaments, das dazu bestimmt ist, der Entwicklung von Arteriosklerose vorzubeugen oder sie zu mildern,
wobei die Zusammensetzung einen In-vitro-Auflösungsgrad, spektralphotometrisch gemessen bei 286 oder 311 nm, entsprechend der im Europäischen Arzneibuch, 3. Ausgabe (oder U.S.P. XXIV) beschriebenen Methode, bei 50 U/min in 500 ml eines 0,1 N HCl-Mediums, bei 37 °C, von:
- 15 bis 25 % von nach 1 Stunde freigesetztem Molsidomin
- 20 bis 35 % von nach 2 Stunden freigesetztem Molsidomin
- 50 bis 65 % von nach 6 Stunden freigesetztem Molsidomin
- 75 bis 95 % von nach 12 Stunden freigesetztem Molsidomin
- > 85 % von nach 18 Stunden freigesetztem Molsidomin
- > 90 % von nach 24 Stunden freigesetztem Molsidomin auf
weist,
wobei der in vivo erreichte Plasmapeak von Molsidomin innerhalb von 2,5 bis 5 Stunden, vorzugsweise innerhalb von 3 bis 4 Stunden nach der Verabreichung der genannten Form vorliegt und einen Wert im Bereich zwischen 25 und 40 ng/ml Plasma aufweist,
wobei die vorgenannte feste orale Zusammensetzung pro Dosierungseinheit, die dazu bestimmt ist, täglich verabreicht zu werden, zwischen 14 und 24 mg Molsidomin umfasst, und
wobei die Behandlung eine Dauer von wenigstens 6 Monaten hat.

2. Verwendung nach Anspruch 1, **dadurch gekennzeichnet, dass** die vorgenannte feste orale Zusammensetzung pro Dosierungseinheit, die dazu bestimmt ist, täglich verabreicht zu werden, 16 mg Molsidomin umfasst.

3. Verwendung nach einem der Ansprüche 1 oder 2, **dadurch gekennzeichnet, dass** die vorgenannte feste orale Zusammensetzung Patienten verabreicht wird, die unter Angina Pectoris leiden.

## Claims

1. Use of molsidomine or one of its pharmaceutically acceptable salts, in the form of a sustained-release solid oral composition effective over 24 hours, for the manufacture of a drug for preventing or attenuating the development of atherosclerosis,
wherein said composition has an in vitro dissolution rate, measured spectrophotometrically at 286 or 311 nm by the method described in the European Pharmacopoeia, 3rd edition (or USP XXIV), at 50 rpm, in 500 ml of a 0.1 N HCl medium, at 37°C, of:
- 15 to 25% of molsidomine released after 1 hour
- 20 to 35% of molsidomine released after 2 hours
- 50 to 65% of molsidomine released after 6 hours
- 75 to 95% of molsidomine released after 12 hours
- >85% of molsidomine released after 18 hours
- >90% of molsidomine released after 24 hours,
the plasma peak of molsidomine obtained in vivo occurring 2.5 to 5 hours, preferably 3 to 4 hours, following the administration of said form, and having a value of between 25 and 40 ng/ml of plasma,
wherein the above-mentioned solid oral composition contains between 14 and 24 mg of molsidomine per dosage unit intended for daily administration, and
wherein said treatment has a duration of at least 6 months.

2. use according to claim 1 or 2, **characterized in that** the above-mentioned solid oral composition contains 16 mg of molsidomine per dosage unit intended for daily administration.

3. Use according to one of claims 1 or 2, **characterized in that** said above-mentioned solid oral composition is administered to patients suffering from angina pectoris.
